Europäisches Patentamt

(19) European Patent Office

Office européen des brevets

(11) Veröffentlichungsnummer: **0 069 958 B2**

(12) **NEUE EUROPÄISCHE PATENTSCHRIFT**

(45) Veröffentlichungstag der neuen Patentschrift:
27.03.91 Patentblatt 91/13

(51) Int. Cl.⁵: **A61K 31/52, A61K 9/54**

(21) Anmeldenummer: 82105982.1

(22) Anmeldetag: 05.07.82

(54) **Verfahren zur Herstellung von magenverträglichen Arzneiformen von Xanthinderivaten.**

(30) Priorität: 10.07.81 DE 3127237

(43) Veröffentlichungstag der Anmeldung:
19.01.83 Patentblatt 83/03

(45) Bekanntmachung des Hinweises auf die
Patenterteilung:
02.04.86 Patentblatt 86/14

(45) Bekanntmachung des Hinweises auf die
Entscheidung über den Einspruch:
27.03.91 Patentblatt 91/13

(84) Benannte Vertragsstaaten:
AT BE CH DE FR GB IT LI LU NL SE

(56) Entgegenhaltungen:
EP-A- 0 005 015
EP-A- 0 011 609
BE-A- 854 640
FR-A- 1 605 467
GB-A- 2 039 737
US-A- 2 738 303
K.Lehmann und D. Dreher "Coating of tablets
and small particles withacrylic resins by fluid
bed technology ", Int.J.Pharm.Techn. and
Prod.Mfr. 2(4) 31-43 (1981).
Eudragit L, Aqueous Dispersion, Data Sheet
(Info LD-2/e) of November 1979
Eudragit L, Aqueous Dispersion, Standards
Sheet Info LD-7/e) of Oktober 1979
Eudragit L, Aqueous Acrylic Resin Dispersion,
Technical Application Pamphlet (Info LD-11/e)
of Mai 1981
Physical Pharmaceutics, E. Shotton and K.
Ridgway, 1974, Clarendon Press, Chapter 9,
page 249.
Goodman and Gilman's The Pharmacological
Basis of Therapeutics, 6th Edition 1980, Macmillan, pages 592-4
Meyler's Side Effects of Drugs, ED. M.N.G.
Dukes, 9th Edition 1980, Excerpta Medica, pages 1-4.
Adverse Drug Reaction Bulletin No. 73, December 1978, Plasma Concentrations in the
prevention and diagnosis of Adverse drug
reactions.
Introduction to Pharmaceutical Dosage
Forms, H.E. Ansel, 1969, Lea and Febiger,
Chapter 14, pages 274-277.

(56) Entgegenhaltungen:
The Theory and Practice of Industrial Pharmacy, Lachman Liebermann and Kaing, 2nd
Edition 1976, pages 456-458.
Eudragit L, Data Sheet (info L-2/e) of 1978.
Eudragit L30D, Prospectus (Info LD-1/e) of
1982 .
Derwent Abstract No. 90018B/50 of J. 54 101
417.
O. Christ et al., Arzneimittel-Forschung 1972,
22 (11) 1933 -1937.
HPMCP Technical Information No. H-16. April
1978.
HPMCP Technical Information No. H-20. January 1979.
CA 94: 90210m Comparison of enteric film
coatings of some synthetic polymers on solid
dosage forms from aqueous and organic coating preparations. (Acta. Pharm. Technol.
1980, 26(3), 201-9).
S.C. Porter "Aqueous film coating: an overview". Pharmaceutical Technology 1979, 3,
55-59.
The theory and practice of Industrial Pharmacy, 2nd Edition, Lea &Febiger Philadelphia
1976, Chapter 12, page 385.

(73) Patentinhaber: HOECHST
AKTIENGESELLSCHAFT
Postfach 80 03 20
W-6230 Frankfurt am Main 80 (DE)

(72) Erfinder: Gleixner, Klaus, Dr.
Beethovenstr. 41
W-6204 Taunusstein (DE)

**Beschreibung**

Xanthinderivate zeichnen sich in der Regel durch sehr unangenehmen, nachhaltig bitteren Geschmack und schlechte Magenverträglichkeit aus. Zur Therapie von Krankheiten insbesondere im Kindesalter, und wegen des stark unterschiedlichen Körpergewichtes sind Arzneiformen erforderlich, die eine individuelle Dosierung zulassen. Normalerweise wird man hierfür Tropfen oder Saft einsetzen. Dies ist jedoch wegen der beschriebenen Eigenschaften vieler Xanthinderivate nicht möglich, da sich der schlechte Geschmack nicht überdecken lässt und die schlechte Magenverträglichkeit zum Teil reflektorisch bedingt ist.

Man hat daher die festen Arzneiformen mit magensaftunlöslichen Filmen überzogen (offengelegte jap. Patentanmeldung Nr. 101417/1979, O. Christ et al., Arzneimittel-Forschung 22 (1972) Heft 11, S. 1933 ff.). Die genannten Schriften beschreiben Arzneiformen, die unter Einsatz von organischen Lösungsmitteln mit magensaft unlöslichen Lackfilmen überzogen werden. Die zu verdampfenden Lösungsmittel stellen eine erhebliche Umweltbelastung dar.

Die oben erwähnte japanische Patentanmeldung beschreibt ein Verfahren zur Herstellung von magensaftresistenten Arzneipellets (Granula) mit dem Xanthinderivat Pentoxifyllin. Diese haben jedoch 2 Nachteile :
1) Zur Herstellung des magensaftresistenten Überzugs aus Basis Hydroxypropylmethylcellulosephthalat wird organisches Lösungsmittel eingesetzt.
2) Es wird sehr viel Hilfsstoff zur Herstellung der Pellets benötigt, so dass 900 mg Granula nur 100 mg Wirkstoff enthalten (ca. 10-12%).

Es wurde nun gefunden, dass man Trägerkügelchen, auf welche Xanthinderivate aufgetragen wurden, mit einem magensaftunlöslichen Überzug versehen kann, wobei bei der Herstellung das magensaftresistente Überzugsmaterial in wässriger Lösung oder Dispersion vorliegt. Diese Granula zeichnen sich durch eine gute Magenverträglichkeit aus.

Die Herstellung und das Überziehen von Granula mit filmbildenden Substanzen ist schwierig, da diese teilweise zu grösseren Agglomeraten verkleben, was zu einer hohen Ausschussquote führt. Überraschenderweise wurde weiterhin gefunden, dass eine gute Magenverträglichkeit auch dann noch gewährleistet ist, wenn die Arzneiform neben der magensaftunlöslichen Granula einen Anteil von bis zu 30% von magensaftlöslichen oder teillöslichen Granula aufweist.

Die Erfindung betrifft daher ein Verfahren zur Vermeidung von Verlusten bei der Herstellung einer Arzneiform auf Basis von Granula mit guter Magenverträglichkeit, enthaltend inerte Trägerkügelchen oder Granula, auf welche Xanthinderivate aufgetragen und weiche mit einem magensaftresistenten Überzug versenen wurden, das dadurch gekennzeichnet, ist dass man die mit einem Xanthinderivat, ausgewählt aus der Gruppe Alkyl- ($\omega$-1)-Oxoalkyl- oder ($\omega$-1)-Hydroxy-alkyldialkylxanthin, dessen Alkyl-, Oxalkyl- bzw. Hydroxyalkylrest jeweils 3 bis 7 C-Atome aufweist und in 1-oder 7-Stellung steht und das in der anderen 1-oder 7-Stellung einen Alkylrest mit 1-12 C-Atomen und in 3-Stellung einen Alkylrest mit : C-Atomen besitzt, beschichteten Trägerkügelchen oder Granula mit Hilfe einer wässrigen Lösung oder wässrigen Dispersion eines magensaftresistenten Überzugsmaterials überzieht, die dabei anfallenden Agglomerate über ein geeignetes Sieb absiebt, in einem Granulator zerkleinert und die dabei entstehenden magensaftresistent überzogenen Granula zumischt, wobei der Anteil an magensaftlösliichen und -teillöslichen Granula bis zu 30% des Gesamtgewichts beträgt und wobei die Granula 20-50% Xanthinderivat 30-75% inertes Trägermaterial und 5-40% magensaftunlösliches Überzugsmaterial enthält : Bevorzugte Ausführungsformen sind Kapseln, Tabletten oder Briefchen, die vorzugsweise bis zu 300 mg Wirkstoff enthalten.

Als Xanthinderivate kommen insbesondere in Betracht Oxohexyldimethylxanthine, bei denen sich der Oxohexyirest entweder in 1-oder 7-Stellung befindet, 1-(4-Hydroxypentyl)- und 1-(5-Hydroxyhexyl)-3,7- dimethylxanthin, 7-(4-Hydroxypentyl)- und 7-(5-Hydroxyhexyl)-1,3- dimethylxanthin, 1-(4-Oxopentyl)-, 1-(2-Methyl-3-oxobutyl)- und 1-(2-Äthyl-3-oxobutyl)-3,7-dimethylxanthin sowie 7-(4-Oxopentyl)-, 7-(2-Methyl-3-oxobutyl)- und 7-(2-Äthyl- 3-oxobutyl)-1,3-dimethyl-xanthin.

Ferner eignen sich Hydroxyalkyl- bzw. Oxoalkyixanthine, die in der 1- oder 7-Stellung statt der Methylgruppe eine Alkylgruppe mit bis zu 4 C-Atomen enthalten, wie das 1-Äthyl-, 1-Propyl, 1-Butyl- und das 1-Isobutyl-3-methyl-7-(5-hydroxyhexyl)-xanthin und die entsprechenden Verbindungen mit den genannten Alkyiresten in 7-Stellung und dem Hydroxylakyl- oder Oxoalkylrest in 1-Stellung. Ganz besonders bevorzugt sind 1-(5-Oxohexyl)-3,7-dimethylxanthin und 3-Methyl-1- (5-oxohexyl)-7-propylxanthin.

Die inerten Trägerkügelchen oder Granula bestehen beispielsweise aus Zucker, Lactose, mikrokristalliner Cellulose oder Kunststoff.

Der magensaftunlösliche Überzug löst sich vorzugsweise oberhalb pH 5,5. Folgende Überzüge bzw. wässrige Lösungen oder Dispersionen, welche zur Herstellung von magensaftresistenten. Überzügen geeignet sind, können erfindungsgemäss angewandt werden :
1. Acrylharz-Lack-Substanzen, wie z.B. Copolymerisat auf Basis von Poly(meth)acrylsäure und Poly(meth)acryisäureestern (Eudragit® L30D).

2. Hydroxypropylcellulosephthalat, feines Pulver (20-30 µm) (z.B. HP 55 F Shin-Etsu Chemicals Co. Tokyo) in Verbindung mit einem Weichmacher (z.B. Triacetin).

3. Eine wässrige Suspension eines Copolymerisats aus 90 Gewichtsteilen Vinylacetat und 10 Gewichsteilen Crotonsäure (z.B. Coating CE 5142, Lieferant BASF).

Bei dem erfindungsgemässen Verfahren werden verfahrensbedingte Verluste fast vollständig vermieden. Prinzipiell ist es möglich, magensaftresistente Granula mit bis zu 30% magensaftlöslichen oder teillöslichen Granula zu mischen. wobei letztere nicht bei der Durchführung des erfindungsgemässen Verfahrens angefallen sind.

Es ist nach dem erfindungsgemässen Verfahren möglich, die gleiche Menge Wirkstoff unter Einsatz von wesentlich weniger Hilfsstoffen zu verabreichen bzw. zu verarbeiten. Die wässrige Lösung oder Dispersion des Fiimmaterials kann die üblichen Zusatzstoffe enthalten.

Die erfindungsgemässe Zubereitung bzw. das erfindungsgemässe Verfahren bietet demnach folgende Vorteile :

1) Wirtschaftliche :

Einsparung von Hilfsstoffen, praktisch keine Materialverluste, keine organischen Lösungsmittel.

2) Für die Herstellung der Granula sind keine aufwendigen Maschinen erforderlich (nur Drageekessel, Rüttelsieb, Granulator und Mischer).

3) Umweltfreundlich, da für die Herstellung der Granula nur wässrige Lösungen oder Dispersionen erforderlich sind.

4) Individuelle Dosierungsmöglichkeit mit geringer Granula-Menge, was besonders in der Kinderpraxis und insbesondere bei Kindern mit z. B. peripheren Durchblutungsstörungen auf diabetischer, entzündlicher sowie funktioneiler Basis wichtig ist.

Die Dosierung richtet sich nach dem Körpergewicht. Beim Erwachsenen sind für die Therapie 100-200 mg Wirksubstanz bis 3x täglich zu verabreichen, bei Kindern 25-100 mg, je nach Körpergewicht. Häufig werden Granula in Hart-Gelatinekapsein abgefüllt. Werden die erfindungsgemässen Granula (Stampfvolumen : 0,85 g/ml) abgefüllt, so lässt sich die Normdosis von 100 mg Wirksubstanz in einer Kapsel der Gr. 1 unterbringen, für die Granula gem. offengelegter apanischer Patentanmeidung Nr. 101417/1979 ist mindestens eine Kapsel der Grösse 00 erforcartich, d.h. das Füllvolumen reicht wahrsrheinlich nicht aus. Die Einnahme einer Kapsel des Formats 00 ist aber für Menschen, insbesondere für Kinder, praktisch nicht mehr zumutbar. Selbst wenn man die Dosis auf zwei Kapseln aufteilt, ist immer noch die Kapsel Grösse 0 erforderlich.

Die Aufteilung der Normaldosis auf mehrere Kapseln birgt zusätzlich die Gefahr. dass unterdosiert wird, weil die Patienten nur 1 Kapsel einnehmen. Nach dem erfindungsgemässen Verfahren lassen sich folgende Wirkstoffmengen in Kapseln unterbringen :

| Dosis mg/Kapsel Wirkstoff | Kapselformat |
|---|---|
| 200 | 00 |
| 100 | 1 |
| 75 | 2 |
| 50 | 3 |
| 25 | 4 |

Die Granula lassen sich auch in Portionspeutel abfüllen, oder nach Vermischen mit werteren Hilfsstoffen zu Tabletten verpressen. Mit den erfindüngsgemässen Granula lässt sich alse eine individueile Therapie durchführen unter Einsatzl von ansprechenden Arzneiformen, die nach einem umweltfreundlichen, material- und kostensparenden Verfahren hergestellt werden.

Aus der erfindungsgemäss hergestelten Arzneiform wird der Wirkstoff verzögert freigesetzt. Je nach Anteil der löslichen bzw. teillöslichen Granula wird im Magen bis zu 30% Wirkstoff freigesetztl während der restliche Teil verzögert im Darmbereich abgegeben wird.

Die nachfolgenden Beispiele erläutern die Erfindung :

Beispiele

1. 100 g Zuckerkügelchen werden im laufenden Dragierkessel mit einem 70%igen Zuckersirup, der zusätzlich arabisches Gummi und Talkum enthält, angefeuchtet. Auf die feuchten Kügelchen streut man nach und nach eine Purvermischung aus

100 g 1-(5-Oxohexyl)-3,7-dimethylxanthin

25 g Talkum und

10 g Siliciumdioxid.

Das Endgewicht der überzogenen Zuckerkügelchen liegt bei etwa 350 g. Nach Trocknung der Granula werden diese im Dragierkessel mit einer ausreichenden Menge einer 15%igen wässrigen Polyacrylharz-Dispersion (Eudragit L 30 D), die zusätlich 1,5% Polyäthylenglykol, 1% Polyvinylpyrrolidon, 3% Talkum und 1% Titandioxid enthält (Trockensubstanz etwa 50 g), besprüht. Das Gesamtgewicht liegt bei etwa 400 g. Anschliessend siebt man die Agglomerate über ain 1,5 mm Sieb ab und zerkleinert sie über ein 1,6 mm Sieb. Von den zerkleinerten Agglomeraten werden dem resistenten Granulat bis zu 30% zugesetzt

2. 100 g 1-(Hexyl)-3,7-dimethylxanthin werden wie unter 1 beschrieben zu Granula verarbeitet.

3. 100 g 1-.(5-Hydroxyhexyl)-3,7-dimethyl-xanthin werden wie unter 1 beschrieben auf Mannitol-Kügelchen aufgetragen und zu Granula verarbeitet.

4. 100 g 1-Propyl-3-methyl-7-(5-hydroxyhexyl)-xanthin werden wie unter 1 beschrieben auf ein im Pelletierteller hergestelltes Lactose-Granulat aufdragiert und mit einer 10%igen wässrigen Suspension auf Basis Vinylaretat/Crotonsäure überzogen und weiterverarbeitet wie unter 1 angegeben.

## Ansprüche

1. Verfahren zur Vermeidung von Verlusten bei der Herstellung einer Arzneiform auf Basis von Granula mit guter Magenverträglichkeit, enthaltend inerte Trägerkügeichen oder Granula, auf welche Xanthinderivate aufgetragen und welche mit einem magensaftre- sistenten Überzug versehen wurden, dadurch gekennzeichnet, dass man die mit einem Xathinderivat, ausgewählt aus der Gruppe Alkyl-, ($\omega$-1)-Oxoalkyl- oder ($\omega$-1)-Hydroxy-alkyldialkylxanthin, dessen Alkyl, Oxalkyl- bzw. Hydroxyalkylrest jeweils 3 bis 7 C-Atome aufweist und in 1- oder 7-Stellung steht und das in der anderen 1-oder 7-Stellung einen Alkylrest mit 1-12 C-Atomen und in 3-Stellung einen Alkylrest mit 1-4 C-Atomen besitzt, beschichteten Trägerkügelchen oder Granula mit Hilfe einer wässrigem Lösung oder wässrigen Dispersion eines magensaftresistenten Überzugsmaterials überzieht, die dabei anfallenden Agglomerate über ein geeignetes Sieb absiebt, in einem Gränulator zerkleinert und die dabei entstehenden magensaftlöslichen bzw. -teillöslichen Granula den magensaftresistent überzogenen Granula zumischt, wobei der Anteil an magensaftlöslichen und -teillöslichen Granula bis zu 30% des Gesamtgewichts beträg 30% und wobei die Granula 20-50% Xanthinderivat, 30-75% inertes Trägermaterial und 5-40% magensaftunlösliches Überzugsmaterial enthält.

2. Verfahren gemäss Anspruch 1, dadurch gekennzeichnet, dass man das Gemisch in eine Kapsel oder ein Briefchen einfüllt oder, gegebenenfalls zusammen mit weiteren Hilfsstoffen, zu Tabletten verpresst.

3. Verfahren gemäss Anspruch 1, dadurch gekennzeichnet, dass man eine Kapsel, Tablette oder Briefchen mit bis zu 300 mg Xanthinderivat herstellt.

4. Verfahren gemäss einem oder mehreren der Ansprüche 1 bis 3, dadurch gekennzeichnet, dass die Xanthinverbindung eine Oxoalkyl- oder Hydroxyalkylverbindung ist, in der der Alkylrest in der 1- oder 7-Stellung 1 bis 4 C-Atome enthält und vorzugsweise in 7-Stellung steht.

5. Verfahren nach einem oder mehreren der Ansprüche 1 bis 4, dadurch gekennzeichnet, dass in der Xanthinverbindung in 3-Stellung Methyl steht.

6. Verfahren nach Anspruch 5, dadurch gekennzeichnet, dass in der Xanthinverbindung die ($\omega$-1)-Hydroxyalkylgruppe 6 C-Atome hat.

7. Verfahren nach einem ader mehreren der Ansprüche 1 bis 6, dadurch gekennzeichnet, dass das Xanthinderivat das 1-(5-Oxohexyl)-3,7-dimethylxanthin oder das 1-(5-Oxohexyl)-3-methyl-7-propylxanthin ist.

8. Verfahren nach Anspruch 1, 2 oder 3, dadurch gekennzeichnet, dass das Xanthinaerivat das 1-Hexyl-3,7-dimethylxanthin ist.

## Claims

1. Process for avoiding waste during preparing of a pharmaceutical form based on granules having a good compatibility with the stomach, containing inert carrier pellets or granules to which xanthine derivatives have been applied and which have been coated with a gastric juice-resistant coating, characterized in that the carrier pellets or granules which have been coated with a xanthine derivative selected from the group of an alkyl- ($\omega$-1)-oxoalkyl- or ($\omega$-1)-hydroxyalkyl-dialkylxanthine, the alkyl, oxoalkyl or hydroxyalkyl radical whereof each has from 3 to 7 carbon atoms and is in the 1- or 7-position and which derivative carries in the other 1- or 7-position

4

an alkyl radical having from 1 to 12 carbon atoms and in the 3-position an alkyl radical having from 1 to 4 carbon atoms, are coated with an aqueous solution or aqueous dispersion of a gastric juice-resistant coating material, the agglomerates obtained are sieved on an appropriate sieve, are crushed in a granulator and the granules thus obtained which are soluble or partially soluble in gastric juice, are admixed to the granules provided with a gastric juice-resistant coating, the portion of granules soluble or partially soluble in gastric juice amounting up to 30% of the total weight, and that the granules contain from 20 to 50% of xanthine derivative, from 30 to 75% of inert carrier material and from 5 to 40% of gastric juice-insoluble coating material.

2. Process according to claim 1, characterized in that mixture is filled in a capsule or a small envelope or, if desired with further adjuvants, is compressed into tablets.

3. Process according to claim 1, characterized in that a capsule, a tablet or a small envelope is produced, each containing up to 300 mg of xanthine derivative.

4. Process according to one or more of claims 1 to 3, characterized in that the xanthine compound is an oxoalkyl or hydroxy-alkyl compound in which the alkyl radical in 1- or 7-position contains from 1 to 4 carbon atoms and is preferably in 7-position.

5. Process according to one or more of claims 1 to 4, characterized in that methyl is present in the 3-position of the xanthine compound.

6. Process according to claim 5, characterized in that the ($\omega$-1)-hydroxy-alkyl group of the xanthine compound has 6 carbon atoms.

7. Process according to one or more of claims 1 to 6, characterized in that the xanthine derivative is the 1-(5-oxohexyl-3,7-dimethylxanthine or the 1-(5-oxohexyl)-3-methyl-7-propylxanthine.

8. Process according to claim 1, 2 or 3, characterized in that the xanthine derivative is the 1-hexyl-3,7-dimethylxanthine.

## Revendications

1. Procédé pour fruite presque complément des partes pour la préparation d'une forme pharmaceutique à base de granules à tolérance bon de la part de l'estomac, contenant de petites billes porteuses ou granules inertes, sur lesquels des dérives de xanthine ont été appliqués et qui ont été pourvus d'un enrobage résistant au suc gastrique, caractérisé par le fait que l'on enrobe les petites billes porteuses ou granules revêtus d'un dérivé de xanthine choisi parmi une alkyl-, une ($\omega$-1)-oxo-alkyl- ou une ($\omega$-1)-hydroxy-alkydialkylxanthine, dont le radical alkyle, oxalkyle ou hydroxy-alkyle présente chaque fois de 3 à 7 atomes de carbone et se trouve en position 1 ou 7 et qui possède à l'autre position 1 ou 7 un radical alkyle ayant 1-12 atomes de carbone et en position 3 un radical alkyle ayant 1-4 atomes de carbone, à l'aide d'une solution aqueuse ou d'une dispersion aqueuse d'un produit d'enrobage résistant au suc gastrique, tamisé sur un tamis approprié les agglomérats formés à cette occasion, les fragmente dans un granulateur et mélange les granules solubles ou partiellement solubles dans le suc gastrique ainsi obtenus avec les granules pourvus d'un enrobage résistant au suc gastrique, la proportion de granules solubles et partiellement solubles dans le suc gastrique constituant jusqu'à 30% du poids total, et les granules contenant 20-50% de dérivé déxanthine, 30–75% de substance porteuse inerte et 5-40% de produit d'enrobage insoluble dans le suc gastrique.

2. Procédé selon la revendication 1, caractérisé par le fait que l'on conditionne le mélange dans une gélule ou dans un cachet, ou qu'on le comprime en comprimés éventuellement conjointement avec d'autres produits auxiliaires.

3. Procédé selon la revendication 1, caractérisé par le fait que l'on prépare une gélule, un comprimé ou un cachet, contenant jusqu'à 300 mg de dérivé de xanthine.

4. Procédé selon l'une ou plusieurs des revendications 1 à 3, caractérisé par le fait que le composé de xanthine est un composé oxo-alkylique ou hydroxy-alkylkique dans lequel le radical alkyle en position 1 ou 7 contient de 1 à 4 atomes de carbone et se trouve de préférence en position 7.

5. Procédé selon l'une ou plusieurs des revendications 1 à 4, caractérisé par le fait que dans le composé de xanthine un groupe méthyle se trouve en position 3.

6. Procédé selon la revendication 5, caractérisé par le fait que le composé xanthique le groupe ($\omega$-1)-hydroxy-alkyle possède 6 atomes de carbone.

7. Procédé selon l'une ou plusieurs des revendications 1 à 6, caractérisé par le fait que le dérivé de xanthine est la 1-(5-oxo-hexyl)-3,7-diméthylxanthine ou la 1-(5-oxo-hexyl)-3-méthyl-7-propylxanthine.

8. Procédé selon la revendication 1, 2 ou 3 caractérisé par le fait que le dérivé de xanthine est la 1-héxyl-3,7-diméthylxanthine.